# EUROPÄISCHE PATENTANMELDUNG

(11) **EP 4 085 903 A1**
(43) Veröffentlichungstag der Anmeldung: **09.11.2022**
(21) Anmeldenummer: 21172815.9
(22) Anmeldetag: 07.05.2021
(51) Int. Cl.: A61K 9/20, A61K 9/28, A61K 31/00, A61P 37/00

(54) **MINITABLETTE ENTHALTEND LOSARTAN FÜR PÄDIATRISCHE ANWENDUNGEN**

(71) Anmelder: Midas Pharma GmbH, 55218 Ingelheim (DE)
(72) Erfinder: Breitkreutz, Jörg, 45721 Haltern am See (DE); Elezaj, Valentine, 40227 Düsseldorf (DE)
(74) Vertreter: Drescher, Christian

(57) **Zusammenfassung**

Die vorliegende Erfindung betrifft Minitabletten für pädiatrische Anwendungen enthaltend Losartan oder eines seiner pharmazeutisch annehmbaren Salze.

## Beschreibung

Losartan ist der internationale Freiname (International nonproprietary name, INN) für 2-Butyl-4-chlor-1-[[2'-(1H-tetrazol-5-yl)[1,1'-biphenyl]-4-yl]methyl]-1H-imidazol-5-methanol.

Losartan blockiert die Interaktion von Angiotensin II und seinem Rezeptor und wird hauptsächlich zur Behandlung von Bluthochdruck, Herzinsuffizienz, ischämischer peripherer Durchblutungsstörung, Myokardischämie (*Angina pectoris*), diabetischer Neuropathie und Glaukom sowie zur Verhinderung des Fortschreitens der Herzinsuffizienz nach Myokardinfarkt eingesetzt. Zudem ist Losartan zur Behandlung der essenziellen Hypertonie zugelassen, und zwar sowohl bei Erwachsenen als auch bei Kindern und Jugendlichen im Alter von 6 bis 18 Jahren.

Eine vorteilhafte Wirkung von Losartan wurde zudem bei der Behandlung von Erkrankungen wie beispielsweise dem *Marfan* Syndrom, der Glasknochenkrankheit (*Osteogenesis Imperfecta,* OI) oder auch fibrotischen Erkrankungen wie der Schmetterlingskrankheit (*Epidermiolysis Bullosa,* EB) aufgezeigt. Viele dieser Krankheiten treten in der Regel schon bei Kindern auf und eine Behandlung muss daher bereits im Kindesalter beginnen.

Eine für die pädiatrische An wendung geeignete Dosierungsform enthaltend Losartan mussfolglich die Anforderung erfüllen, dass die Dosis des Therapeutikums mit den Kindern wächst, d.h. in möglichst kleinen Schritten angepasst werden kann.

Eine für die pädiatrische Anwendung vorgesehene Darreichungsform von Losartan-Kalium in Form von 2,5 mg/ml Pulver sowie Lösungsmittel zur Herstellung einer Suspension zum Einnehmen wurde 2014 unter dem Handelsnamen LORZAAR^{®} zugelassen.

Diese Formulierung weist neben dem Wirkstoff Losartan eine Vielzahl von weiteren Bestandteilen auf, nämlich ein Pulver enthaltend Mikrokristalline Cellulose (E 460), Lactose-Monohydrat, Vorverkleisterte Maisstärke, Magnesiumstearat pflanzlich (E 572) sowie Opadry WHITE bestehend aus Hyprolose (E 463), Hypromellose (E 464) und Titandioxid (E 171) sowie ein Lösungsmittel enthaltend Mikrokristalline Cellulose (E 460), Carmellose-Natrium, Citronensäure, Wasser, Xanthangummi (E 415), Methyl-4-hydroxybenzoat (E 218), Natriumdihydrogenphosphat Monohydrat, Kaliumsorbat, Carrageen, Calciumsulfat, Natriumphosphat, Glycerol, Beeren-Zitronenaroma, süß, Propyl-4-hydroxybenzoat (E 216), Natriumcitrat, Saccharin-Natrium, Sorbitol (E 420) und eine entschäumende AF-Emulsion bestehend aus Wasser, Dimeticon, Glycerolmono/di [-palmitat, -stearat], Macrogolstearat und Macrogol.

Gemäß der LORZAAR^{®}-Fachinformation beträgt die empfohlene tägliche Initialdosis Losartan-Kalium für Patienten im Alter von 6 bis 18 Jahren 0,7 mg/kg, was bei einem Körpergewicht zwischen 20 kg und 50 kg einer Tagesdosis von 14 mg bis 35 mg Wirkstoff entspricht.

Die LORZAAR^{®}-Suspension weist mehrere Nachteile auf. Um sie stabil zu halten, müssen Konservierungsmittel zugesetzt werden. Zudem ist eine exakte Dosierung recht umständlich und ohne weitere Hilfsmittel kaum möglich. Suspensionen neigen zur Sedimentation und müssen daher bei mehrmaliger Verwendung immer wieder aufgeschüttelt werden. Zur Geschmacksmaskierung des äußerst bitter schmeckenden Losartans werden sowohl die Viskositätserhöher Carrageenan und Xanthan-Gummi als auch mehrere Süß- bzw. Aromastoffe zugegeben.

Eine Aufgabe der vorliegenden Erfindung kann in der Bereitstellung einer für die pädiatrische Anwendung geeigneten oralen Arzneiform enthaltend Losartan gesehen werden, welche die Nachteile der im Stand der Technik bekannten Arzneiform vermeidet.

Die Aufgabe wird durch die im Folgenden beschriebenen Minitabletten enthaltend Losartan gelöst.

Die für pädiatrische Anwendungen geeigneten Minitabletten gemäß der vorliegenden Erfindung enthalten Losartan oder eines seiner pharmazeutisch annehmbaren Salze. Durch den geringen Wirkstoffgehalt bieten die erfindungsgemäßen Minitabletten den Vorteil, dass sie sowohl zur genauen individuellen Einstellung als auch zur inkrementellen Erhöhung der Dosis geeignet sind, also eine flexible Anpassung der Dosierung an das sich ändernde Körpergewicht von Kindern erlauben, und zwar durch die Gabe ganzer Einheiten.

Beispielsweise beträgt bei einem Kind mit 20 kg Körpergewicht die zu verabreichende tägliche Dosis von 0,7 mg Losartan pro kg Körpergewicht 14 mg Losartan, was 14 Minitabletten ä 1 mg, 10 Minitabletten à 1,4 mg oder 7 Minitabletten à 2 mg entspricht.

Einer Erhöhung des Körpergewichtes um nur 2 kg kann einfach durch die Gabe einer zusätzlichen Minitablette à 1,4 mg Rechnung getragen werden.

Zur erleichterten Dosierung können Minitabletten gemäß der vorliegenden Erfindung über individuell einstellbare Dosierspender verabreicht werden. Ein Aspekt der Erfindung betrifft daher ein Kit umfassend 50 bis 2.000 erfindungsgemäße Losartan-Minitabletten sowie einen Dosierspender.

Die erfindungsgemäßen Minitabletten weisen ein Verhältnis von Oberfläche zu Volumen von 2:1 bis 6:1 mm⁻¹ auf.

Die Masse der Mintabletten beträgt vorzugsweise 3 mg bis 15 mg, bevorzugt 4 mg bis 10 mg, besonders bevorzugt 5 mg bis 9 mg.

Darüber hinaus weisen bevorzugte erfindungsgemäße Minitabletten einen Durchmesser sowie eine Höhe von 1 mm bis 3 mm, weiter bevorzugt von 1,5 mm bis 2,5 mm auf. Dadurch entsteht ein weiterer Vorteil, nämlich der einer verbesserten Schluckbarkeit dieser Vielzahl klein dimensionierter und daher leicht schluckbarer Einheiten. Zudem können Minitabletten mit einem Durchmesser von bis zu 3mm bereits bei Kindern ab zwei Jahren sicher angewendet werden.

Ein weiterer Vorteil der erfindungsgemäßen Minitabletten ist die im Vergleich zur Suspension verbesserte physikalische und chemische Stabilität, so dass keine Konservierungsmittel zugesetzt werden müssen.

Die pharmazeutischen Minitabletten der vorliegenden Erfindung enthalten den Wirkstoff Losartan vorzugsweise in Form eines seiner Salze, bevorzugt dem Kaliumsalz, wobei jede Einheit eine Einzeldosis von 0,5 mg bis 4 mg, bevorzugt 1 mg bis 3 mg des Wirkstoffs enthält.

Losartan muss wegen der kleinen Dimensionen von Minitabletten in relativ hohen Anteilen enthalten sein. Dazu kommt, dass Minitabletten ein vergrößertes Oberfläche-zu-VolumenVerhältnis aufweisen. Da Losartan eine schlechte Fließfähigkeit aufweist und bei der Formulierung stark zum Verkleben der Werkzeuge neigt, stellt die Entwicklung von Minitabletten mit hohem Losartananteil eine besondere Herausforderung dar, zudem bei deren Herstellung auch auf Grund der kleineren Matrizenöffnungen besonders auf eine gute Fließfähigkeit der Formulierungen geachtet werden muss.

Die pharmazeutischen Minitabletten der vorliegenden Erfindung enthalten außer dem Wirkstoff einen oder mehrere Hilfsstoffe, beispielsweise einen oder mehrere Füllstoffe, ein oder mehrere Bindemittel, einen oder mehrere Zerfallsbeschleuniger oder/und einen oder mehrere Schmiermittel.

Pharmazeutisch akzeptable Füllstoffe gemäß der vorliegenden Erfindung umfassen Cellulosederivate wie mikrokristalline Cellulose (MCC), Methylcellulose, Stärken, Zuckeralkohole wie Mannitol, Sorbitol oder Xylitol, Acrylatpolymere und -copolymere, Dextrate, Dextrin, Dextrose, Maltodextrin, Pektin und Gelatine. Bevorzugter Füllstoff ist silifizierte mikrokristalline Cellulose. Die Gesamtmenge an Füllstoffen liegt im Bereich von 20 Gew.-% bis 80 Gew.-%, vorzugsweise im Bereich von 30 Gew.-% bis 60 Gew.-%, bezogen auf das Gesamtgewicht der Zusammensetzung.

Pharmazeutisch akzeptable Bindemittel gemäß der vorliegenden Erfindung umfassen Vinylpyrrolidon-Vinylacetat Copolymere (Copovidon), Hydroxypropylcellulose (HPC), Dihydroxypropylcellulose, Hydroxyethylcellulose, Polymethacrylate, Natriumalginat, Polyvinylpyrrolidon (Povidon), vorverkleisterte Stärke und Mischungen davon. Bevorzugtes Bindemittel ist Copovidon. Die Gesamtmenge an Bindemittel liegt im Bereich von 0,5 Gew.-% bis 10 Gew.-%, vorzugsweise im Bereich von 3 Gew.-% bis 7 Gew.-%, bezogen auf das Gesamtgewicht der Zusammensetzung.

Pharmazeutisch akzeptable Zerfallsbeschleuniger gemäß der vorliegenden Erfindung umfassen Crospovidon, Croscarmellose, Carboxymethylcellulose, Carboxymethylstärke und Mischungen davon. Bevorzugter Zerfallsbeschleuniger ist Crospovidon. Die Gesamtmenge an Zerfallsbeschleuniger liegt im Bereich von 1 Gew.-% bis 10 Gew.-%, vorzugsweise im Bereich von 1 Gew.-% bis 4 Gew.-% bezogen auf das Gesamtgewicht der Zusammensetzung.

Pharmazeutisch akzeptable Schmiermittel gemäß der vorliegenden Erfindung umfassen Talkum, Magnesiumstearat, Calciumstearat, Natriumstearat, Stearinsäure, Hexandisäure, Natriumstearylfumarat, Glycerinfumarat und Mischungen davon. Bevorzugte Schmiermittel sind Magnesiumstearat und Talkum. Die Gesamtmenge an Schmiermittel liegt im Bereich von 1 Gew.-% bis 5 Gew.-%, vorzugsweise im Bereich von 2 Gew.-% bis 4 Gew.-%, bezogen auf das Gesamtgewicht der Zusammensetzung.

Weiter können die erfindungsgemäßen Minitabletten optional sprödbrüchige Hilfsstoffe enthalten, um die Druckfestigkeit der Tablette beim Verpressen zu erhöhen. Pharmazeutisch akzeptable sprödbrüchige Hilfsstoffe gemäß der vorliegenden Erfindung umfassen Calciumphosphat, Tricalciumcitrat, Mannitol, Saccharose, Xylitol und Lactitol sowie Mischungen davon. Bevorzugter sprödbrüchiger Hilfsstoffist Dicalciumphosphat Anhydrat. Die Gesamtmenge an sprödbrüchigen Hilfsstoffen, sofern vorhanden, liegt im Bereich von 10 Gew.-% bis 30 Gew.-%, vorzugsweise im Bereich von 15 Gew.-% bis 25 Gew.-% bezogen auf das Gesamtgewicht der Zusammensetzung.

Die aus dem Stand der Technik bekannte LORZAAR^{®}- Suspension enthält neben Süßstoffen die Viskositätserhöher Carrageenan und Xanthangummi. Diese Hilfsstoffe tragen zur Geschmacksmaskierung bei, indem sie die Viskosität der Suspension erhöhen und damit die Zeit verlängern, die der Wirkstoff benötigt, um zu den Geschmacksrezeptoren auf der Zunge zu gelangen. Anders ausgedrückt, wird dadurch der Kontakt des Wirkstoffs zu den Geschmacksrezeptoren auf der Zunge vor dem Schlucken verkürzt. Diese Art der Geschmacksmaskierung ist allerdings flüssigen Arzneiformen wie Suspensionen vorbehalten und daher bei den erfindungsgemäßen festen Arzneiformen nicht anwendbar.

Die Erfinder der vorliegenden Anmeldung haben herausgefunden, dass für pädiatrische Formulierungen des nicht nur äußerst unangenehm schmeckenden, sondern zugleich sehr gut löslichen Losartans die Erreichtung einer physikalischen Barriere um das Losartan zur optimalen Geschmacksmaskierung vorteilhaft ist. Die erfindungsgemäßen Minitabletten können daher eine physikalische Barriere aufweisen, welche die Freisetzung des Losartans verzögert und den sehr bitteren Geschmack idealerweise so lange unterdrückt, bis die Minitablette vom Patienten heruntergeschluckt ist.

Erfindungsgemäß besteht die physikalische Barriere aus einem Überzug, der die Freisetzung des Wirkstoff anfänglich verzögert. Der Überzug besteht bevorzugterweise aus einem pharmazeutisch akzeptablen, wasserlöslichen Filmbildner. Bevorzugte Filmbildner gemäß der vorliegenden Erfindung sind wasserlösliche Polymere wie Hydroxypropylmethylcellulose (HPMC).

Zudem können die Befilmungen optional Weichmacher enthalten, beispielsweise Polyethylenglykol (PEG).

Filmbildner und Weichmacher werden bevorzugt in einem Verhältnis von 20 zu 1 bis 5 zu 1, bevorzugt von 12 zu 1 bis 8 zu 1 eingesetzt.

Für pädiatrische Anwendungen genügt in der Regel die Verzögerung der Freisetzung des Losartans aus der Minitablette um eine Minute. Es wurde gefunden, dass die Verzögerung der Wirkstofffreisetzung mit der Dicke der Befilmung korreliert. So resultierte ein Auftrag von 5 mg/cm² in einer Verzögerung der Wirkstofffreisetzung um eine Minute. Bei einem Auftrag der doppelten Menge, d.h. von 10 mg/cm² erfolgte die Freisetzung des Losartans aus den Minitabletten entsprechend erst nach zwei Minuten in nenneswerter Menge.

Ein weiterer Aspekt der vorliegenden Erfindung betrifft daher Losartan enthaltende Minitabletten sowie einen Überzug, wobei die Freisetzung des Losartans in nenneswerter Menge frühestens nach 30 Sekunden nach Verabreichung der Arzneiform, bevorzugt frühestens nach zwischen 1 Minute und 5 Minuten besonders bevorzugt nach zwischen 2 Minuten und 5 Minuten erfolgt.

Zur Geschmacksmaskierung bzw. -verbesserung der erfindungsgemäßen Minitabletten können auch Komplexbildner wie beispielsweise Cyclodextrine oder Ionenaustauscher, Lipoproteine mit einer hohen Affinität zu den Geschmacksrezeptoren wie beispielsweise Adenosinmonophosphat sowie den Geschmack verbessernde Mittel wie Süßungsmittel und Aromastoffe zugegeben werden.

Die erfindungsgemäßen Minitabletten können nach einem Verfahren hergestellt werden, in dem in einem ersten Schritt ein Granulat aus Losartan sowie einem oder mehreren Füllmittel hergestellt wird. In einem zweiten Schritt wird das im ersten Schritt erhaltene Granulat mit einem oder mehreren Bindemitteln sowie einem oder mehreren Zerfallsbeschleunigern vermischt. Optional kann das Bindemittel auch bereits im Granulat enthalten sein. In einem dritten Schritt werden zu der im zweiten Schritt erhaltenen Mischung eines oder mehrere Schmiermittel dazugegeben. Im vierten Schritt erfolgt die Verpressung der Mischung zu Tablettenkernen und in einem fünften Schritt werden die Tablettenkerne beschichtet. In den Beispielen sind weitere Details zum Herstellungsverfahren genannt.

Die erfindungsgemäßen Minitabletten sind besonders für pädiatrische Anwendungen wie der Behandlung der essenziellen Hypertonie, dem *Marfan* Syndrom, Osteogenesis imperfecta sowie fibrotischen Erkrankungen geeignet, da die erfindungsgemäßen Minitabletten sowohl eine geringe Initialdosis als auch eine Anpassung der Dosis an das Körpergewicht in kleinen Einheiten gewährleisten. Eine bevorzugte Verwendung besteht in der Behandlung der Erkrankung Epidermolysis Bullosa, wobei die Minitabletten von 1 mg bis 2 mg, bevorzugt von 1,3 mg bis 1,7 mg Losartan Kalium enthalten.

Eine weitere bevorzugte Ausführungsform der vorliegenden Erfindung betrifft eine Minitablette für pädiatrische Anwendungen mit einem Verhältnis von Oberfläche zu Volumen von 2:1 bis 6:1 mm⁻¹, enthaltend Losartan oder eines seiner pharmazeutisch annehmbaren Salze, einen oder mehrere Hilfsstoffe sowie einen Überzug, wodurch die Freisetzung des Wirkstoffs nach der Einnahme um 30 Sekunden oder länger verzögert wird.

Eine weitere bevorzugte Ausführungsform der vorliegenden Erfindung betrifft eine Minitablette für pädiatrische Anwendungen mit einem Verhältnis von Oberfläche zu Volumen von 2:1 bis 6:1 mm⁻¹, enthaltend 0,5 mg bis 4 mg Losartan oder eines seiner pharmazeutisch annehmbaren Salze, 20 Gew.-% bis 80 Gew.-% eines oder mehrerer Füllstoffe sowie einen Überzug enthaltend einen oder mehrere Filmbidlner, wodurch die Freisetzung des Wirkstoffs nach der Einnahme um zwischen 1 und 5 Minuten verzögert wird.

Eine weitere bevorzugte Ausführungsform der vorliegenden Erfindung betrifft eine Minitablette für pädiatrische Anwendungen mit einem Verhältnis von Oberfläche zu Volumen von 2:1 bis 6:1 mm⁻¹, einem Gesamtgewicht von 3 mg bis 15 mg, enthaltend 1 mg bis 3 mg Losartan oder eines seiner pharmazeutisch annehmbaren Salze, 20 Gew.-% bis 80 Gew.-% eines oder mehrerer Füllstoffe, 0,5 Gew.-% bis 10 Gew.-% eines oder mehrerer Bindemittel, 1 Gew.-% bis 10 Gew.-% eines oder mehrerer Zerfallsbeschleuniger, 1 Gew.-% bis 5 Gew.-% eines oder mehrerer Schmiermittel sowie einen Überzug enthaltend ein oder mehrere Polymere sowie optional einen oder mehrere Weichmacher, wodurch die Freisetzung des Wirkstoffs nach der Einnahme zwischen 1 und 5 Minuten verzögert wird.

Eine weitere bevorzugte Ausführungsform der vorliegenden Erfindung betrifft eine Minitablette zur Behandlung von Epidermolysis bullosa mit einem Verhältnis von Oberfläche zu Volumen von 2:1 bis 6:1 mm⁻¹, einem Gesamtgewicht von 5 mg bis 8 mg und einem Durchmesser sowie einer Höhe von jeweils 1,5 mm bis 2,5 mm, enthaltend 1,3 mg bis 1,7 mg Losartan Kalium, 30 Gew.-% bis 60 Gew.-% mikrokristalline Cellulose, 3 Gew.-% bis 7 Gew.-% Copovidon, 1 Gew.-% bis 4 Gew.-% Crospovidon, 2 Gew.-% bis 4 Gew.-% eines Gemisches aus Magnesiumstearat und Talkum sowie einen Überzug bestehend aus Hydroxypropylmethylcellulose (HPMC), wodurch die Freisetzung des Wirkstoffs nach der Einnahme um zwischen 2 und 4 Minuten verzögert wird.

### BEISPIEL 1

In einem ersten Schritt werden Losartan Kalium (42,7%) und silifizierte mikrokristalline Cellulose (57,3%) gemischt und mittels Walzenkompaktor ein Trockengranulat hergestellt.

Die Partikelgröße des Granulats ist dabei einer der Faktoren, welche dessen Fließfähigkeit beeinflussen. Generell steigt die Fließfähigkeit mit steigender Partikelgröße an, wobei Partikel größer 250 µm als frei fließend, Partikel kleiner als 100 µm als kohäsiv und Partikel kleiner 10 µm als extrem kohäsiv gelten.

Tabelle 1 zeigt auf, dass die mittlere Partikelgröße mit steigender spezifischer Kompaktierkraft (SKK) zunimmt. Bei einem mit einer SKK von 6 kN/cm kompaktierten Granulat sind nur 10% aller Partikel kleiner als 100 µm. Die Partikelgrößenbestimmung wurde durchgeführt mit einem Camsizer XT, wobei eine Druckluftdispergierung durchgeführt wurde (x-jet, Dispergierdruck: 30 kPa, digitalen Bildverarbeitung mit Zwei-Kamera-System; Messbereich 1 µm - 3 mm).

**Tabelle 1: Partikeldurchmesser des Granulats**

| | SSK = 2 kN/cm | SSK = 4 kN/cm | SSK = 6 kN/cm |
|---|---|---|---|
| Mittlerer Partikeldurchmesser [µm] | | | |
| D₁₀ | 59 ± 1 | 75 ± 1 | 101 ± 14 |
| D₅₀ | 634 ± 12 | 799 ± 8 | 832 ± 12 |
| D₉₀ | 1135 ± 4 | 1183 ± 4 | 1204 ± 4 |

Eine bevorzugte Ausführungsform der vorliegenden Erfindung betrifft daher ein Granulat enthaltend Losartan, dadurch gekennzeichnet, dass nur 10% aller Partikel (D₁₀) kleiner als 100µm sind.

Ein weiterer Parameter für das Fließverhalten ist der sog. Hausner-Faktor. Das Fließverhalten ist dabei umso besser, je kleiner der *Hausner*-Faktor ist, siehe Tab. 2. Eine 3-fach-Bestimmung des Hausner-Faktors gemäß Ph.Eur. 2.9.36 für bei einer SKK von 6 kN/cm hergestellten Granulaten ergab einen *Hausner*-Faktor von 1,33 ± 0,03, was einem mäßigem Fließverhalten entspricht, unter den gegebenen schwierigen Rahmenbedingungen aber als ausreichend anzusehen ist.

**Tabelle 2: Hausner-Faktor gemäß Ph.Eur. 2.9.36**

| Fließverhalten | Hausner-Faktor | Kompressibilitätsindex |
|---|---|---|
| ausgezeichnet | 1,00-1,11 | 1-10% |
| Gut | 1,12-1,18 | 11-15% |
| zufriedenstellend | 1,19-1,25 | 16-20% |
| mäßig | 1,26-1,34 | 21-25% |
| schlecht | 1,35-1,45 | 26-31% |
| sehr schlecht | 1,46-1,59 | 32-37% |
| ungenügend | > 1,60 | > 38% |

Eine bevorzugte Ausführungsform der vorliegenden Erfindung betrifft daher die Herstellung einer Losartan enthaltenden Minitablette aus einem Granulat mt einem Hausner-Faktor von 1,34 oder niedriger.

In einem zweiten Schritt wird zunächst das in Schritt 1 erhaltene Granulat mit dem Copovidon und dem Crospovidon vermischt. Anschließend werden Magnesiumstearat und Talkum zugegeben und die resultierende Mischung mit einer Rundläuferpresse tablettiert, wobei bei Bedarf Siebschritte zwischengeschaltet werden können. Die Zusammensetzung der resultierenden Minitabletten ist in Tabelle 3 dargestellt.

**Tabelle 3. Zusammensetzung der Minitabletten**

| Material | Funktion | Menge [mg] pro Einheit | Menge [%] |
|---|---|---|---|
| Losartan Kalium | Wirkstoff | 2,50 | 38.46 |
| Silifizierte Mikrokristalline Cellulose | Füllmittel | 3,34 | 51.54 |
| Copovidon | Bindemittel | 0,33 | 5.00 |
| Crospovidon | Zerfallsbeschleuniger | 0,13 | 2.00 |
| Magnesiumstearat | Schmiermittel | 0,13 | 2.00 |
| Talkum | | 0,07 | 1.00 |
| | Gesamt: | 6,50 | 100 |

Um dem anschließenden Beschichtungsschritt standzuhalten, müssen die Minitabletten eine ausreichende Bruchfestigkeit aufweisen. Die Bruchfestigkeit nimmt mit steigendem Pressdruck, bei dem die Minitabletten tablettiert werden, zu. Die erfindungsgemäßen Minitabletten wiesen dabei schon bei einem Pressdruck von 100 MPa eine ausreichende Bruchfestigkeit von etwa 0,9 MPa auf. Eine weitere bevorzugte Ausführungsform der vorliegenden Erfindung betrifft daher Minitabletten enthaltend Losartan, die eine mittlere Bruchfestigkeit von 0,9 MPa oder höher aufweisen.

Auch der Abrieb (Friabilität) kann als Parameter zur Beurteilung der mechanischen Eigenschaften von Minitabletten herangezogen werden. Daher wurde eine Prüfung auf Friabilität der erfindungsgemäßen Minitabletten gemäß Ph.Eur. 2.9.7 durchgeführt. Die Minitabletten zeigten dabei einen Abrieb von 0,14 ± 0,03%, was weit unterhalb der Grenze von 1,0% gemäß Europäischen Arzneibuch liegt. Eine weitere bevorzugte Ausführungsform der vorliegenden Erfindung betrifft daher Minitabletten enthaltend Losartan, die einen Abrieb von weniger als 1%, bevorzugt einen Abrieb von weniger als 0,5%, noch weiter bevorzugt einen Abrieb von weniger als 0,2% aufweisen.

Der durchschnittliche Wirkstoffgehalt erfindungsgemäßer Minitabletten wurde gemäß Ph.Eur. 2.9.40 bestimmt.

**Tabelle 4. Wirkstoffgehalt der Minitabletten (n=10)**

| Mittelwert [%] | s [%] | rsd [%] | Akzeptanzwert | Limit (Ph.Eur. 2.9.40) |
|---|---|---|---|---|
| 100,8 | 4,8 | 4,7 | 11,5 | 15 |

Tabelle 4 zeigt, dass der mittlere Gehalt an Losartan unter Berücksichtigung der Standardabweichung innerhalb des vom Arzeibuch vorgegebenen Akzeptanzwertes liegt.

Die in Schritt 2 erhaltenen unbeschichteten Minitabletten werden in einem dritten Schritt mittels Bottom-Spray in der Wirbelschicht befilmt. Die Beschichtungslösung enthält neben entmineralisiertem Wasser 12 Gew.-% Hydroxypropylmethylcellulose (HPMC) sowie 1,2 Gew.-% Polyethlenglykol (PEG 6000). Nach einem Aufheizschritt folgt der Sprühprozess mit anschließendem Trocknungsschritt. Die Geräteeinstellungen für den Beschichtungsprozess sind in Tabelle 5 dargestellt.

**Tabelle 5: Beschichtungsprozess - Geräteeinstellungen**

| Gerät | Mycrolab-Fluidgerät (Hüttlin, Deutschland) |
|---|---|
| Innendurchmesser der Düse | 0,8 mm |
| Sprühdruck | 1,0 bar |
| Mikroklima | 0,15-0,25 bar |
| Zulufttemperatur | 48°C |
| Zuluftmenge | 13 m³/h |
| Pumprate | 1,3g/min |

Einige Parameter der so hergestellten Minitabletten sind in Tabelle 6 aufgeführt.

**Tabelle 6. Abmessungen der Minitabletten (n=20)**

| Durchmesser [mm] | Höhe [mm] | Masse [mg] |
|---|---|---|
| 2,15 ± 0,01 | 2,21 ± 0,03 | 7,96 ± 0,32 |

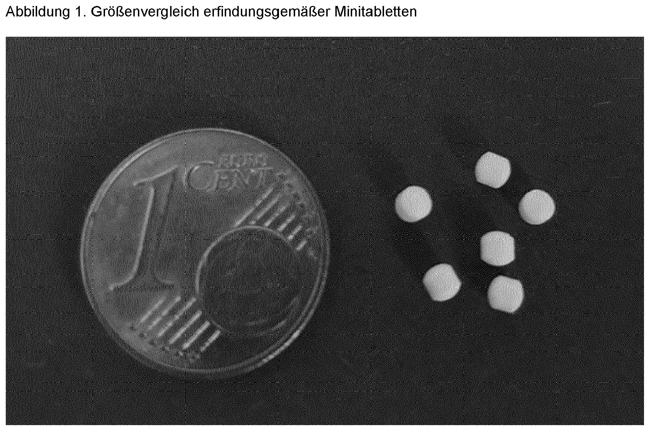

Abbildung 1 zeigt einen Größenvergleich der so hergestellten Minitabletten.

Die Dissolutionsstudien der so hergestellten Minitabletten wurden in Phosphatpuffer pH 6,0 bei 37 ± 0,5°C mit einer Korbapparatur nach Ph.Eur. durchgeführt. Die Wirkstofffreisetzung wurde mittels HPLC mit UV-Vis-Detektor gemessen.

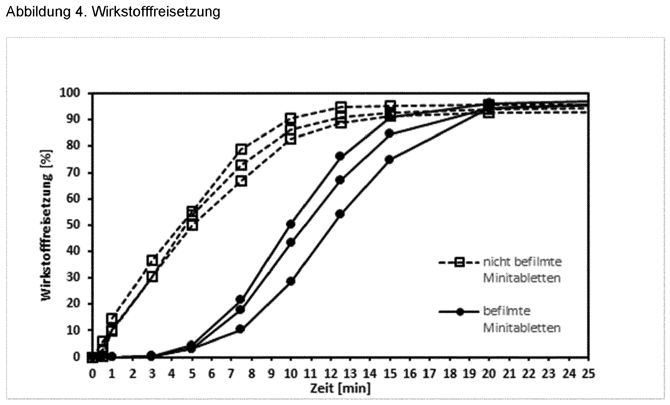

Wie Abbildung 4 zeigt, erfolgt die Freisetzung des Wirkstoffs aus unbefilmten Minitabletten praktisch unmittelbar. Die Freisetzungsprofile befilmter Minitabletten weisen hingegen eine anfängliche Verzögerung der Wirkstofffreisetzung auf. Im Vergleich zu den unbeschichteten Minitabletten kann dadurch der bittere Geschmack von Losartan über den Zeitraum, in dem die Minitabletten im Mund des Patienten bleiben, vermieden werden.

Die anfängliche Verzögerung der Wirkstofffreisetzung nimmt mit zunehmendem Polymerauftrag zu. Eine Massenbelegung von 5 mg/cm² entsprechend einer Schichtdicke der Befilmung von 35 µm bis 45 µm, resultierte in einer Verzögerung der Freisetzung des Losartans von über einer Minute. Bei einer Massenbelegung von rund 10 mg/cm², entsprechend einer Schichtdicke der Befilmung von 75 +/- 10 µm, wurde der Wirkstoff mit einer Verzögerung von über zwei Minuten freigesetzt.

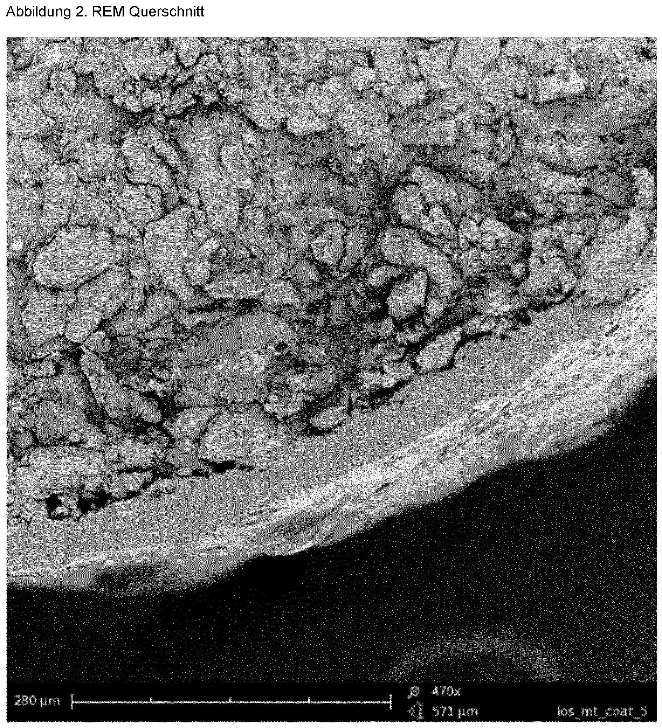

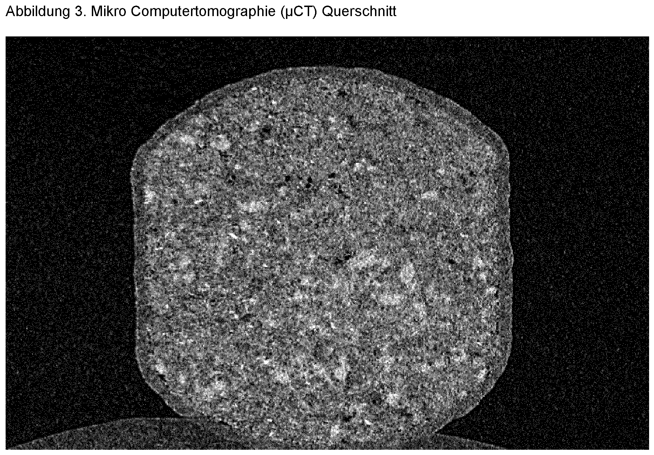

Die REM- Aufnahme eines Querschnittes einer mit 5 mg/cm² Polymer befilmten Minitablette in Abbildung 2 sowie die Mikro-Computertomographie-Aufnahme (µCT) eines Querschnittes einer mit 10 mg/cm² Polymer befilmten Minitablette in Abbildung 3 zeigen jeweils eine gleichmäßige Beschichtung mit einem vollständigen und intakten Film.

Wie aus Abbildung 4 ebenfalls hervorgeht, erfolgt eine über die initiale Verzögerung hinausgehende Retardierung nicht, so dass sowohl aus befilmten als auch unbefilmten Minitabletten nach 20 Minuten gleichermaßen über 90% des Wirkstoffs freigesetzt werden.

Die erfindungsmäßige Beschichtung gewährleistet damit einen geschmacksmaskierenden Effekt, der über die Massenbelegung bzw. Schichtdicke der Befilmung elegant steuerbar ist.

### BEISPIEL 2

**Tabelle 7. Zusammensetzung der Minitabletten**

| Material | Funktion | Menge per MiniTablette | Anteil [%] |
|---|---|---|---|
| Losartan Kalium | Wirkstoff | 1,5 | 27,27% |
| Silifizierte Mikrokristalline Cellulose | Füllmittel | 2,16 | 39,27% |
| Dicalciumphosphat Anhydrat | Sprödbrüchiger Hilfsstoff | 1,3 | 23,64% |
| Copovidon | Bindemittel | 0,26 | 4,73% |
| Crospovidon | Zerfallsbeschleuniger | 0,11 | 2,00% |
| Magnesiumstearat | Schmiermittel | 0,11 | 2,00% |
| Talkum | | 0,06 | 1,09% |
| | Gesamt: | 5.5 mg | 100,00 |

Erfindungsgemäße Minitabletten werden hergestellt, indem in einem ersten Schritt Losartan, silifizierte mikrokristalline Cellulose, Calciumphosphat und Copovidon gemischt werden und ein Trockengranulat mittels Walzenkompaktor hergestellt wird. In einem weiteren Schritt wird das in Schritt 1 erhaltene Trockengranulat mit Crospovidon vermischt. Anschließend werden Magnesiumstearat und Talkum in zwei separaten Mischschritten zugegeben. Die resultierende Mischung wird an einer Rundläuferpresse tablettiert. Die erhaltenen Minitabletten werden mittels Bottom-Spray in der Wirbelschicht befilmt. Zur Zusammensetzung der Sprühlösung siehe Beispiel 1. Nach einem Aufheizschritt folgt der Sprühprozess mit anschließendem Trocknungsschritt.

Die Minitabletten gemäß Beispiel 2 sind wegen des Wirkstoffgehalts besonders zur Anwendung bei der Behandlung der Erkrankung Epidermolysis Bullosa geeignet.

## Patentansprüche

1. Minitablette für pädiatrische Anwendungen enthaltend Losartan oder eines seiner pharmazeutisch annehmbaren Salze, **gekennzeichnet durch** ein Verhältnis von Oberfläche zu Volumen von 2:1 bis 6:1 mm⁻¹.

2. Minitablette für pädiatrische Anwendungen gemäß Anspruch 1, **gekennzeichnet durch** ein Gesamtgewicht von 3 mg bis 15 mg, bevorzugt von 4 mg bis 10 mg, besonders bevorzugt 5 mg bis 8 mg.

3. Minitablette für pädiatrische Anwendungen gemäß Anspruch 1 oder 2, **dadurch gekennzeichnet, dass** sie 0,25 mg bis 4 mg, bevorzugt 1 mg bis 3 mg Losartan oder eines seiner pharmazeutisch annehmbaren Salze enthält.

4. Minitablette für pädiatrische Anwendungen gemäß einem der vorhergehenden Ansprüche, **gekennzeichnet durch** einen Durchmesser sowie eine Höhe von 1 mm bis 3 mm, bevorzugt 1,5 mm bis 2,5 mm.

5. Minitablette für pädiatrische Anwendungen gemäß einem der vorhergehenden Ansprüche, weiterhin umfassend ein oder mehrere Füllmittel ausgewählt aus der Gruppe bestehend aus mikrokristalliner Cellulose (MCC), Methylcellulose, Stärken, Zuckeralkohole wie Mannitol, Sorbitol oder Xylitol, Dextrate, Dextrin, Dextrose, Maltodextrin, Pektin und Gelatine.

6. Minitablette für pädiatrische Anwendungen gemäß einem der vorhergehenden Ansprüche, weiterhin umfassend ein oder mehrere Bindemittel ausgewählt aus der Gruppe bestehend aus Vinylpyrrolidon Vinylacetat Copolymere (Copovidon), Hydroxypropylcellulose (HPC), Dihydroxypropylcellulose, Hydroxyethylcellulose, Polymethacrylate, Natriumalginat, Polyvinylpyrrolidon (Povidon), vorverkleisterte Stärke.

7. Minitablette für pädiatrische Anwendungen gemäß einem der vorhergehenden Ansprüche, weiterhin umfassend ein oder mehrere Zerfallsbeschleuniger ausgewählt aus der Gruppe bestehend aus Crospovidon, Croscarmellose, Carboxymethylcellulose sowie Carboxymethylstärke.

8. Minitablette für pädiatrische Anwendungen gemäß einem der vorhergehenden Ansprüche, weiterhin umfassend ein oder mehrere Schmiermittel ausgewählt aus der Gruppe bestehend aus Magnesiumstearat, Talkum, Calciumstearat, Natriumstearat, Stearinsäure, Hexandisäure, Natriumstearylfumarat, Calciumbehenat, Natriumglycerylbehenat sowie Glycerinfumarat.

9. Minitablette für pädiatrische Anwendungen gemäß einem der vorhergehenden Ansprüche, weiterhin umfassend einen Überzug, **dadurch gekennzeichnet, dass** die Freisetzung des Wirkstoffs Losartan nach der Einnahme um 1 Minute oder länger verzögert wird.

10. Minitablette für pädiatrische Anwendungen gemäß Anspruch 10, wobei der Überzug eines oder mehrere Polymere enthält.

11. Minitablette für pädiatrische Anwendungen gemäß einem der Ansprüche 19 oder 10, wobei die Dicke des Überzugs zwischen 20 µm und 100 µm, bvorzugt zwischen 35 µm und 85 µm beträgt.

12. Minitablette für pädiatrische Anwendungen gemäß einem der vorhergehenden Ansprüche, wobei die pädiatrische Anwendung ausgewählt ist aus der Gruppe bestehend aus der essenziellen Hypertonie, dem *Marfan* Syndrom, Osteogenesis imperfecta sowie fibrotischen Erkrankungen.

13. Minitablette für pädiatrische Anwendungen gemäß Anspruch 13, wobei die fibrotische Erkrankung Epidermolysis bullosa ist und die Minitablette von 1 mg bis 2 mg, bevorzugt 1,3 mg bis 1,7 mg Losartan Kalium enthält.

14. Kit zur individuellen Einstellung der Dosis umfassend einen Dosierspender sowie 50 bis 2.000 Minitabletten für pädiatrische Anwendungen gemäß einem der vorhergehenden Ansprüche.

15. Verfahren zur Herstellung einer Minitablette gemäß einem der Ansprüche 1 bis 14, umfassend die Verfahrensschritte
i. Herstellung eines Granulates aus Losartan mindestens einem oder mehreren Füllmitteln sowie gegebenenfalls weiteren Hilfsmitteln,
ii. Vermischen des im ersten Schritt erhaltene Granulates mit einem oder mehrenen Zefallsbeschleunigern sowie optional einem oder mehrenen Hilfsmitteln,
iii. Vermischen der im zweiten Schritt erhaltenen Mischung mit einem oder mehrenen Schmiermitteln sowie optional einem oder mehrenen Hilfsmitteln,
iv. Verpressung der Mischung zu Minitabletten,
v. optional Beschichtung der Minitabletten
